# EUROPÄISCHE PATENTSCHRIFT

(11) **EP 0 536 189 B1**
(45) Veröffentlichungstag und Bekanntmachung des Hinweises auf die Patenterteilung: **27.12.1995**
(21) Anmeldenummer: 91911221.9
(22) Anmeldetag: 17.06.1991
(51) Int. Cl.: C07C 233/38, C07D 233/16, C11D 1/52, D06M 13/41

(54) **SALZE VON FETTSÄUREAMIDEN UND FETTSÄUREIMIDAZOLINEN**
SALTS OF FATTY-ACID AMIDES AND FATTY-ACID IMIDAZOLINES
SELS D'AMIDES D'ACIDES GRAS ET D'IMIDAZOLINES D'ACIDES GRAS

(30) Priorität: 26.06.1990 DE 4020271
(43) Veröffentlichungstag der Anmeldung: 14.04.1993
(73) Patentinhaber: Henkel Kommanditgesellschaft auf Aktien, 40191 Düsseldorf (DE)
(72) Erfinder: UPHUES, Günter, D-4019 Monheim (DE); PLOOG, Uwe, D-5657 Haan (DE); JESCHKE, Rainer, D-4000 Düsseldorf 13 (DE); WALTENBERGER, Peter, D-5461 Breitscheid-Hollig (DE)
(86) Internationale Anmeldenummer: EP9101121
(87) Internationale Veröffentlichungsnummer: WO9200272

(56) Entgegenhaltungen:
- EP-A- 0 186 967
- EP-A- 0 230 910
- CH-A- 414 665
- JOURNAL OF THE AMERICAN OIL CHEMISTS SOCIETY, Band 60, Nr. 4, April 1983, R.G. BISTLINE, Jr. et al: "Synthesis and properties of fatty imidazolines and their N-(2-aminoethyl) derivatives", Seiten 823-828
- CHEMICAL ABSTRACTS, Band 95, Nr. 9, 31. August 1981, Seite 774, Zusammenfassung Nr. 80815u, Columbus, Ohio, US, R.N. BUTLER et al: "Reactions of fatty acids with amines. Part 3. Thermal reactions of oleic and elaidic acids (cis- and trans-octadec-9-enoic acids) with some 1,2-diamines: ready reversibility of imidazoline formation"
- FETTE, SEIFEN, ANSTRICHMITTEL, Band 74, Nr. 9, September 1972, H.W. ECKERT: "Kondensationsprodukte aus beta-Hydroxyäthyläthylendiamin und Fettsäuren bzw. Fettsäurealkylestern und ihre Anwendung als Textilweichmacher in Feinwaschmitteln"
- FETTE, SEIFEN, ANSTRICHMITTEL, Band 88, Nr. 10, September 1986, M. MÜNZING et al: "Kinetik der Fetthärtung und Vergleich verschiedener Katalysatoren", Seiten 387-391

## Beschreibung

Gegenstand der Erfindung sind Salze von Fettsäureamiden und Fettsäureimidazolinen, erhältlich durch Umsetzung von ungesättigten Fettsäuren, die einen Gehalt von mindestens 40 Mol-% trans-konfigurierter Doppelbindungen aufweisen, oder deren Ester mit Aminen und anschließende Neutralisation mit Säuren sowie die Verwendung der Produkte als Wäscheweichspüler.

Durch Behandlung mit Avivagemitteln kann textilem Fasergut ein weicher, angenehmer Griff verliehen und damit der Warencharakter günstig beeinflußt werden. Ferner dienen das Weichmachen und die damit verbundene Erhöhung der Elastizität bei im Gebrauch befindlichen Textilien dazu, das Tragegefühl zu verbessern und das Material pflegeleichter zu machen. Diese spezielle Form des Avivierens wird meist als Wäschenachbehandlung vom Verbraucher selbst durchgeführt. Als Avivagemittel finden dabei vor allem kationaktive Verbindungen mit längerkettigen Alkylresten wie z. B. Ditalgalkyldimethylammoniumchlorid **[J.Am.Oil.Chem.Soc. 66, 718 (1989)]**, quaternierte Fettsäuretriethanolaminester **[EP 0 302 567]** oder Kondensationsprodukte von Fettsäuren mit Aminoalkanolaminen **[Fette, Seif., Anstrichmitt., 74, 527 (1972)]** Verwendung. Textilien, die mit Avivagemitteln der genannten Art behandelt werden, verfügen zwar über einen guten Weichgriff, weisen jedoch häufig eine unerwünschte Hydrophobie auf, die sich in einer schlechten Benetzbarkeit oder Wasseraufnahme der behandelten Gewebe äußert.

Aufgabe der Erfindung war es demnach, Avivagemittel zu entwickeln, die in der Lage sind, Textilien sowohl einen guten Weichgriff, als auch eine hohe Hydrophilie zu verleihen.

Gegenstand der Erfindung sind Salze von Fettsäureamiden und Fettsäureimidazolinen, erhältlich durch Umsetzung von ungesättigten Fettsäuren mit 16 bis 22 Kohlenstoffatomen und einem Gehalt von mindestens 40 Mol-% trans-konfigurierter Doppelbindungen oder deren Ester mit Glycerin oder aliphatischen Alkoholen mit 1 bis 4 Kohlenstoffatomen mit Aminen der Formeln **(I)** oder **(II)**, 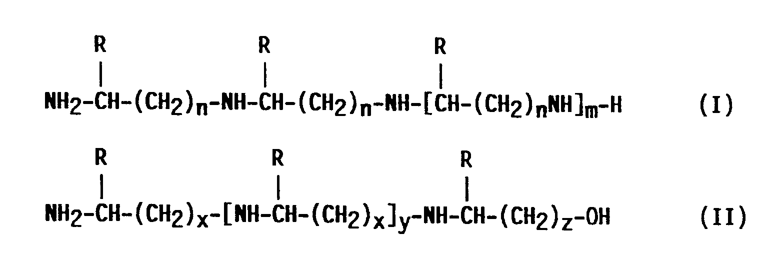
in denen R für Wasserstoff oder einen Methylrest sowie m, n, x, y und z unabhängig voneinander für 0 oder Zahlen von 1 bis 5 stehen, und anschließende Neutralisation der Reaktionsprodukte mit organischen oder anorganischen Säuren.

Die Erfindung beruht auf der Erkenntnis, daß Umsetzungsprodukte von Fettsäuren, die zu mindestens 40 Mol-% mit trans-konfigurierten Doppelbindungen vorliegen, mit Aminen der genannten Art, Textilien bei der avivierenden Behandlung nicht nur einen guten Weichgriff, sondern auch eine überraschend hohe Hydrophilie verleihen.

Als Fettsäuren für die Herstellung der erfindungsgemäßen Fettsäureamide kommen insbesondere die Elaidinsäure sowie elaidinsäurereiche technische Fettsäurefraktionen in Betracht, die einen Gehalt an Fettsäuren mit trans-konfigurierten Doppelbindungen von mindestens 40 Mol-% aufweisen.

Als Ausgangsmaterial für die Herstellung der Salze der Fettsäureamide und Fettsäureimidazoline kommen weiterhin Ester der vorgenannten Fettsäuren mit Alkoholen mit 1 bis 4 Kohlenstoffatomen in Betracht. Typische Beispiele sind Ethyl-, n-Propyl-, i-Propyl-, n-Butyl- oder insbesondere Methylester. In einer besonderen Ausführungsform der Erfindung können die Fettsäuren ferner als Voll- oder Partialester des Glycerins vorliegen.

Zur Herstellung der ungesättigten Fettsäuren mit einem Gehalt an trans-konfigurierten Doppelbindungen von mindestens 40 Mol-% oder deren Ester kann man von Linolsäure oder deren Ester ausgehen, die in Gegenwart von modifizierten Nickel-Katalysatoren selektiv zu Gemischen einfach ungesättigter Fettsäuren mit hohem Elaidinsäuregehalt ("trans-Gehalt") oder deren Ester hydriert werden. Eine andere Möglichkeit besteht darin, cis-Octadecensäure (Ölsäure) oder deren Ester in Gegenwart von Selen oder Salpetersäure zur trans-Octadecensäure (Elaidinsäure) oder deren Ester zu isomerisieren. Derartige Verfahren zur Herstellung von Elaidinsäure und Elaidinsäureestern sind seit langem bekannt und z. B. in **Fette, Seif., Anstrichmitt., 88, 387 (1987)** oder **J.Am.Chem.Soc., 79, 4765 (1967)** beschrieben.

Wie in der Fettchemie üblich, können zur Herstellung der ungesättigten Fettsäuren mit einem Gehalt an trans-konfigurierten Doppelbindungen von mindestens 40 Mol-% oder deren Ester auch technische Gemische von Fettsäuren oder deren Ester eingesetzt werden, die auf Basis natürlicher Fette und Öle erhalten werden. In diesem Fall wird der trans-Gehalt von den in den technischen Gemischen enthaltenen gesättigten Anteilen begrenzt. Vorzugsweise geht man daher von Produkten aus, die einen hohen Gehalt an Linol- oder Ölsäure und einen geringen Anteil gesättigter Verbindungen aufweisen, wie z. B. Sonnenblumenöl oder Rüböl.

Salze von Fettsäureamiden und Fettsäureimidazolinen mit besonders guten anwendungstechnischen Eigenschaften werden erhalten, wenn man technische Elaidinsäure- oder Elaidinsäureestergemische mit einem Gehalt von mindestens 40 Mol-%, vorzugweise mindestens 50 Mol-%, insbesondere mehr als 60 Mol-% Elaidinsäure, mit Aminen der Formeln **(I)** oder **(II)** umsetzt und die Reaktionsprodukte anschliessend mit anorganischen oder organischen Säuren neutralisiert.

Als Amine kommen Oligoamine wie z. B. Diethylentriamin, Triethylentetramin, Tetraethylenpentamin, Di-n-propylentriamin, Tri-n-propylentetramin und Di-i-propylentriamin oder Aminoalkylalkanolamine wie z. B. Aminoethylethanolamin, Amino-n-propylpropanolamin oder Amino-i-propylpropanolamin in Betracht. Bei Einsatz von Aminoalkanolaminen kann, wenn auch untergeordnet, Esterbildung zwischen der Hydroxylfunktion des Amins und der Fettsäure stattfinden.

Fettsäureamide mit besonders vorteilhaften anwendungstechnischen Eigenschaften werden erhalten, wenn man von Aminen der Formel **(I)**, in der R für Wasserstoff, n für 1 oder 2 und m für 0 oder 1 steht, oder Aminen der Formel **(II)** ausgeht, in der R für Wasserstoff, x und z für 1 und y für 0 steht.

Gegenstand der Erfindung ist weiterhin ein Verfahren zur Herstellung von Salzen von Fettsäureamiden und Fettsäureimidazolinen, dadurch gekennzeichnet, daß man ungesättigte Fettsäuren mit 16 bis 22 Kohlenstoffatomen und einem Gehalt von mindestens 40 Mol-% trans-konfigurierter Doppelbindungen oder deren Ester mit Glycerin oder aliphatischen Alkoholen mit 1 bis 4 Kohlenstoffatomen mit Aminen der Formeln **(I)** oder **(II)**, 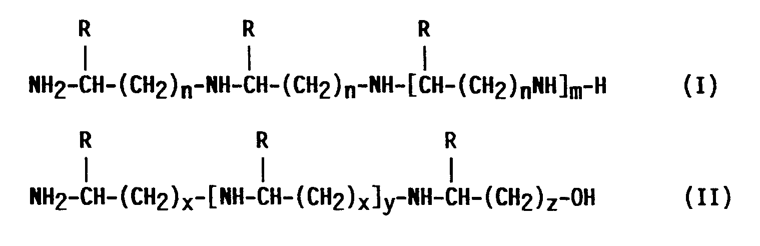
in denen R für Wasserstoff oder einen Methylrest sowie m, n, x, y und z unabhängig voneinander für 0 oder Zahlen von 1 bis 5 stehen, umsetzt und die Reaktionsprodukte anschließend mit organischen oder anorganischen Säuren neutralisiert.

Werden freie Fettsäuren in die Reaktion eingesetzt, findet mit den Aminen eine Amidierung statt. Die Reaktionspartner können dabei in einem molaren Verhältnis von Fettsäure zu Amin von 1 : 1 bis 5 : 1, vorzugsweise 1,5 : 1 bis 3 : 1 eingesetzt werden.

Werden Ester der ungesättigten Fettsäuren eingesetzt, findet mit den Aminen eine Aminolyse statt. Für die Einsatzmengen der Reaktionspartner gelten die oben genannten Bedingungen. Bei der Aminolyse von Di- oder Triglyceriden mit Aminen beziehen sich die molaren Einsatzmengen auf die molare Menge der in den Fettsäureglycerinestern vorhandenen Fettsäurereste.

Die Amidierung der Fettsäuren mit den Aminen kann bei einer Temperatur von 150 bis 220°C durchgeführt werden. Eine optimale Reaktionsgeschwindigkeit wird erreicht, wenn die Reaktion im Bereich von 180 bis 200°C durchgeführt wird. Die Umsetzung wird dabei solange fortgesetzt, bis das Reaktionsprodukt eine Säurezahl kleiner 5 aufweist.

Die Aminolyse der Fettsäureester mit den Aminen kann bei niedrigeren Temperaturen von 80 bis 220, vorzugsweise 80 bis 150°C durchgeführt werden. Durch Zusatz von 0,1 bis 0,5 Gew.-% eines basischen Katalysators, z. B. Natriummethylat, kann die Reaktionsgeschwindigkeit gesteigert werden. Der bei der Umsetzung gebildete Alkohol kann, wenn dies gewünscht wird, durch Destillation entfernt werden.

Nach der Amidierung oder Aminolyse wird das alkalische Rohprodukt neutralisiert. Hierzu kommen absolute, vorzugsweise jedoch wäßrige, 50 bis 70 gew.-%ige Säuren aus der Gruppe, die von Phosphorsäure, Ameisensäure, und Milchsäure gebildet wird, in Betracht. Vorzugsweise wird die Neutralisation mit wäßriger 70 gew.-%iger Glykolsäure, Phosphoriger Säure oder Essigsäure durchgeführt.

Bei der avivierenden Behandlung von Textilien, wie beispielsweise Gewebe, Gewirke oder Garne, verleihen die Fett- säureamide den Geweben einen guten Weichgriff und eine hohe Hydro- philie. Sie eignen sich daher als Avivagemittel für Textilien Avivage- und Wäscheweichspülmittel sowie zur Herstellung von Wäscheweichspülmitteln.

Die folgenden Beispiele sollen den Gegenstand der Erfindung näher erläutern, ohne ihn darauf einzuschränken.

### Beispiele

### I. Einsatzstoffe

Fettsäure A wurde durch Selektivhydrierung von technischer Sonnenblumenfettsäure mit einem Gehalt von 62 Gew.-% Linolsäure in Gegenwart von modifizierten Nickelkatalysatoren gemäß dem in **Fette, Seif., Anstrichmitt., 88, 387 (1987)** beschriebenen Verfahren hergestellt und wies nach der Hydrierung einen trans-Gehalt von 62 Gew.-% auf. Zur Herstellung der Fettsäuren B - E mit trans-Gehalten von 50 bis 31 Gew.-% wurde die Fettsäure A mit technischer Ölsäure oder Stearinsäure abgemischt. Die Kenndaten zu den eingesetzten Fettsäuren sind in Tab.1 zusammengefaßt.

Die Produkte auf Basis technischer Fettsäuren mit einem trans-Gehalt von mindestens 40 Mol-% sind erfindungsgemäß; Produkte auf Basis technischer Fettsäuren mit geringerem trans-Gehalt (D,E) dienen dem Vergleich.

Das technische Triglyceridgemisch F wurde durch Selektivhydrierung von neuem, ölsäurereichem Rüböl nach dem oben geschilderten Verfahren gewonnen. Zum Vergleich wurde hydrierter Rindertalg (G) in die Reaktion eingesetzt. Die Kenndaten der eingesetzten Triglyceride sind in Tab.1 zusammengefaßt.

**Tab.1**

| Einsatzstoffe | | | | |
|---|---|---|---|---|
| Einsatzstoff | Säurezahl | Iodzahl | Verseifungszahl | trans-Gehalt Gew.-% |
| A | 197,7 | 89,5 | | 62 |
| B | 199,5 | 72,3 | | 50 |
| C | 198,2 | 90,2 | | 50 |
| D | 199,0 | 91,2 | | 31 |
| E | 202,5 | 45,5 | | 32 |
| F | | 87,0 | 198 | 72 |
| G | | 1,0 | 198 | 0 |

### II. Herstellungsbeispiele

### Beispiel 1:

**Kondensation von Fettsäuren mit Aminoethylethanolamin**. In einem 1 l-Dreihalskolben mit Rührer, Innenthermometer und Destillationsaufsatz wurden 426 g (1,5 Mol) der technischen Fettsäure A mit hohem Elaidinsäuregehalt vorgelegt, auf 80°C erwärmt und mit 104 g (1,0 Mol) Aminoethylethanolamin versetzt. Die Reaktionsmischung wurde in 3 h auf 200°C erhitzt und 1 h bei gleicher Temperatur nachgerührt, wobei 26 g Kondensationswasser abgeschieden wurden. Nach Abkühlung auf 20°C wurden 499 g einer hellgelben festen Masse erhalten, die eine Säurezahl von 2,4 und 1,6 Gew.-% titrierbaren Stickstoff aufwies (Produkt 1).

100 g des Reaktionsproduktes, 12,6 g Eisessig und 450,4 g Wasser wurden gemeinsam unter Rühren auf 80°C erwärmt und 30 Minuten nachgerührt. Nach Abkühlung auf 20°C wurden 560 g einer feinteiligen, weißen Dispersion mit einem Feststoffgehalt von 20 Gew.-% erhalten (Produkt 1*).

### Beispiele 2 und 3, Vergleichsbeispiele 1 und 2:

Beispiel 1 wurde unter Einsatz technischer Fettsäuren mit hohem (Fettsäuren B, C; Beispiele 2 und 3) oder geringem Elaidinsäuregehalt (Fettsäuren D,E Vergleichsbeispiele 1 und 2) wiederholt (Produkte 2, 3 und 2*, 3* sowie V1, V2 und V1*, V2*)

### Beispiel 4:

100 g des Reaktionsproduktes aus Beispiel 1 wurden geschmolzen, bei 80°C mit 8,8 g phosphoriger Säure neutralisiert und mit 435 g Wasser von 85°C verdünnt. Nach einer Nachrührzeit von 30 min wurde rasch auf 20°C abgekühlt. Es wurden 540 g einer feinteiligen Dispersion mit einem Feststoffgehalt von 20 Gew.-% erhalten (Produkt 4*)

### Beispiel 5:

**Kondensation von Fettsäuren mit Diethylentriamin**. In einer Apparatur analog Beispiel 1 wurden 419,5 g (1,5 Mol) der technischen Fettsäure A mit hohem Elaidinsäuregehalt vorgelegt, auf 80°C erwärmt und mit 77,2 g (0,75 Mol) Diethylentriamin vermischt. Die Mischung wurde in 3,5 h auf auf 200°C erhitzt und 1 h bei gleicher Temperatur nachgerührt, wobei 28 g Kondensationswasser abgeschieden wurden. Unter Druckverminderung auf 2,5 · 10³ Pa wurde 2 h auf 200°C erhitzt, wobei weitere 13 g Wasser abgeschieden wurden. Nach Abkühlung auf 20°C wurden 455 g einer festen, spröden Masse erhalten, die eine Säurezahl von 3,4 und 2,2 Gew.-% titrierbaren Stickstoff aufwies. Die UV-spektroskopische Analyse ergab einen Imidazolingehalt von 0,198 Mol/100 g (Produkt 5).

100 g des Reaktionsproduktes wurden geschmolzen, bei 95°C mit 9,5 g Eisessig versetzt und anschließend mit 438 g Wasser von 85°C verdünnt. Es wurden 545 g einer gelartigen Paste mit einem Feststoffgehalt von 20 Gew.-% erhalten (Produkt 5*)

### Beispiele 6 und 7, Vergleichsbeispiele 3 und 4:

Beispiel 5 wurde unter Einsatz technischer Fettsäure mit hohem (Fettsäuren B und C, Beispiele 6 und 7) oder geringem Elaidinsäuregehalt (Fettsäuren D und E, Vergleichsbeispiele 3 und 4) wiederholt (Produkte 6, 7 und 6*, 7* sowie V3, V4 und V3* und V4*).

### Beispiel 8:

**Aminolyse von Triglyceriden mit Aminoethylethanolamin.** In einer Apparatur analog Bsp.1, jedoch Rückflußkühler anstelle des Destillationsaufsatzes, wurden 302 g (0,35 Mol) des technischen Triglyceridgemisches auf Basis Rüböl (F) vorgelegt, auf 80°C erwärmt und mit 55,4 g (0,53 Mol) Aminoethylethanolamin versetzt und auf 100°C erhitzt. Die Reaktion wurde unter Rühren fortgesetzt, bis der Gehalt an titrierbarem Stickstoff auf 1,4 Gew.-% abgesunken war. Danach wurde auf 90°C abgekühlt und die Reaktion durch Zugabe von 72,4 g 70 gew.-%iger Glykolsäure abgebrochen (Produkt 8).

80 g der bei 20°C festen Masse wurden mit 320 g Wasser bei 80 bis 85°C zu einer feinteiligen Dispersion vermischt und anschließend rasch auf 20°C abgekühlt (Produkt 8*).

### Vergleichsbeispiel V5:

Beispiel 8 wurde unter Einsatz von 300 g (0,35 Mol) gehärtetem Rindertalg (G) wiederholt. Es wurden die Produkte V5 und V5* erhalten.

### Beispiel 9:

Beispiel 8 wurde unter Einsatz von 37,1 g (0,35 Mol) Aminoethylethanolamin wiederholt. Die Reaktion wurde bei einem Gehalt an titrierbaren Stickstoff von 1,05 Gew.-% durch Zusatz von 29,1 g Glykolsäure abgebrochen. Es wurden die Produkte 9 und 9* erhalten.

### Vergleichsbeispiel V6:

Beispiel 9 wurde unter Einsatz von 300 g (0,35 Mol) gehärtetem Rindertalg (G) wiederholt. Es wurden die Produkte V6 und V6* erhalten.

### III. Anwendungstechnische Beispiele

Durch wiederholtes Waschen gehärtetes Baumwollgewebe (Molton) wurde unter Anwendung des Foulardverfahrens mit den Produkten der Beispiele 1 bis 9 sowie der Vergleichbeispiele 1 bis 6 behandelt. Dabei galten folgende Vorgaben:

| | |
|---|---|
| Konzentration | 30 g/l der 20 gew.-%igen Produkte |
| Flottenaufnahme | ca. 80 Gew.-% (bezogen auf trockenes Gewebe) |
| Trocknung | 3 min bei 180°C |

Von den ausgerüsteten Prüfgeweben wurden die Wiederbenetzbarkeit und der Weichgriff beurteilt. Zur Prüfung der Wiederbenetzbarkeit diente der Steighöhentest nach **DIN 53 924**, wobei die Wassersteighöhe in mm nach 1 min bewertet wurde. Die Beurteilung des Weichgriffs erfolgte subjektiv durch 6 erfahrene Personen, die auf einer Skala von 0 = hart und rauh bis 6 = weich und voluminös Noten vergeben konnten. Die Testergebnisse sind in Tab.2 zusammengefaßt.

Unter den gleichen Bedingungen wurde Baumwoll-Frottiergewebe ausgerüstet. Die Beurteilung des Weichgriffs zeigte dem Moltongewebe vergleichbare Ergebnisse.

Zur weiteren Prüfung der Wiederbenetzbarkeit wurde ein Wassertropfen auf die Prüfgewebe aufgebracht. Mit Ausnahme der Vergleichsbeispiele, bei denen Salze von Fettsäureamiden mit höherem Gehalt an gesättigten Fettsäuren eingesetzt wurden, wurde der Wassertropfen spontan vom Gewebe aufgenommen.

**Tab.2**

| Wiederbenetzbarkeit und Weichgriff | | | |
|---|---|---|---|
| Produkt | Fettsäure | Steighöhe mm | Griffnote |
| 1* | A | 13 | 4,5 |
| 2* | B | 10 | 5 |
| 3* | C | 15 | 4,5 |
| 4* | A | 10 | 5 |
| 5* | A | 14 | 5,5 |
| 6* | B | 5 | 5,5 |
| 7* | C | 10 | 5 |
| 8* | F | 15 | 6,0 |
| 9* | F | 22 | 5,0 |
| V1* | D | 20 | 3,5 |
| V2* | E | 0 | 5,5 |
| V3* | D | 0 | 5,5 |
| V4* | E | 25 | 4 |
| V5* | G | 0 | 5,5 |
| V6* | G | 0 | 5,0 |

## Patentansprüche

1. Salze von Fettsäureamiden und Fettsäureimidazolinen, erhältlich durch Umsetzung von ungesättigten Fettsäuren mit 16 bis 22 Kohlenstoffatomen und einem Gehalt von mindestens 40 Mol-% trans-konfigurierter Doppelbindungen oder deren Ester mit Glycerin oder aliphatischen Alkoholen mit 1 bis 4 Kohlenstoffatomen mit Aminen der Formeln **(I)** oder **(II)**, 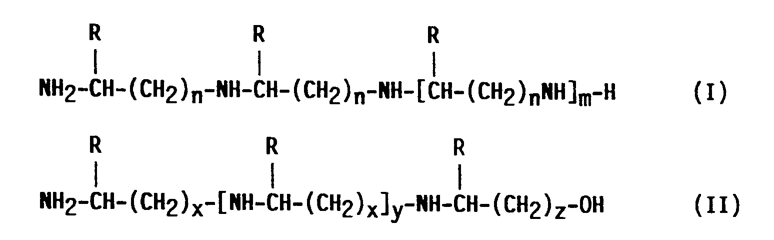 in denen R für Wasserstoff oder einen Methylrest sowie m, n, x, y und z unabhängig voneinander für 0 oder Zahlen von 1 bis 5 stehen, und anschließende Neutralisation der Reaktionsprodukte mit Säuren.

2. Salze nach Anspruch 1, dadurch gekennzeichnet, daß man technische Elaidinsäure oder deren Ester mit Aminen der Formeln **(I)** oder **(II)** umsetzt.

3. Salze nach mindestens einem der Ansprüche 1 und 2, dadurch gekennzeichnet, daß in Formel **(I)** R für Wasserstoff, n für 1 oder 2 und m für 0 oder 1 steht.

4. Salze nach mindestens einem der Ansprüche 1 und 2, dadurch gekennzeichnet, daß in Formel **(II)** R für Wasserstoff, x und z für 1 und y für 0 steht.

5. Verfahren zur Herstellung von Salzen von Fettsäureamiden und Fettsäureimidazolinen, dadurch gekennzeichnet, daß man ungesättigte Fettsäuren mit 16 bis 22 Kohlenstoffatomen und einem Gehalt von mindestens 40 Mol-% trans- konfigurierter Doppelbindungen oder deren Ester mit Glycerin oder aliphatischen Alkoholen mit 1 bis 4 Kohlenstoffatomen mit Aminen der Formeln **(I)** oder **(II)**, 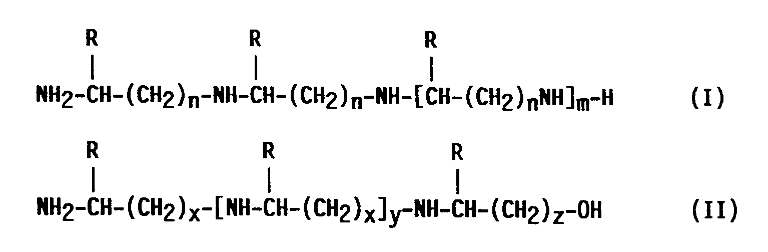 in denen R für Wasserstoff oder einen Methylrest sowie m, n, x, y und z unabhängig voneinander für 0 oder Zahlen von 1 bis 5 stehen, umsetzt und die Reaktionsprodukte anschließend mit anorganischen oder organischen Säuren neutralisiert.

6. Verfahren nach Anspruch 5, dadurch gekennzeichnet, daß man technische Elaidinsäure oder deren Ester mit Aminen der Formeln **(I)** oder **(II)** umsetzt.

7. Verfahren nach mindestens einem der Ansprüche 5 und 6, dadurch gekennzeichnet, daß man Amine der Formel **(I)** einsetzt, in der R für Wasserstoff, n für 1 oder 2 und m für 0 oder 1 steht.

8. Verfahren nach mindestens einem der Ansprüche 5 und 6, dadurch gekennzeichnet, daß man Amine der Formel **(II)** einsetzt, in der R für Wasserstoff, x und z für 1 und y für 0 steht.

9. Verfahren nach mindestens einem der Ansprüche 5 bis 8, dadurch gekennzeichnet, daß man die Umsetzung der ungesättigten Fettsäuren oder deren Ester mit den Aminen in einem molaren Verhältnis von 1 : 1 bis 5 : 1 durchführt.

10. Verfahren nach mindestens einem der Ansprüche 5 bis 9, dadurch gekennzeichnet, daß man die Umsetzung der ungesättigten Fettsäuren oder deren Ester mit den Aminen bei Temperaturen von 80 bis 220°C durchführt.

11. Verfahren nach mindestens einem der Ansprüche 5 bis 10, dadurch gekennzeichnet, daß man die Neutralisation der Reaktionsprodukte mit Säuren aus der Gruppe, die von Phosphorsäure, Phosphoriger Säure, Ameisensäure, Essigsäure, Glykolsäure und Milchsäure gebildet wird, durchführt.

12. Verwendung von Salzen von Fettsäureamiden und Fettsäureimidazolinen nach mindestens einem der Ansprüche 1 bis 4 als Avivage- und Wäscheweichspülmittel.

13. Verwendung von Salzen von Fettsäureamiden und Fettsäureimidazolinen nach mindestens einem der Ansprüche 1 bis 4 zur Herstellung von Avivage- und Wäscheweichspülmittel.

14. Verwendung von Salzen von Fettsäureamiden und Fettsäureimidazolinen, erhältlich nach dem Verfahren nach mindestens einem der Ansprüche 5 bis 11 als Wäscheweichspülmittel.

15. Verwendung von Salzen von Fettsäureamiden und Fettsäuremimidazoline, erhältlich nach dem Verfahren nach mindestens einem der Ansprüche 5 bis 11 zur Herstellung von Avivage- und Wäscheweichspülmittel.

## Claims

1. Salts of fatty acid amides and fatty acid imidazolines obtainable by reaction of unsaturated fatty acids containing 16 to 22 carbon atoms and at least 40 mol-% transconfigured double bonds or esters thereof with glycerols or C₁₋₄ aliphatic alcohols with amines corresponding to formula **(I)** or **(II)** 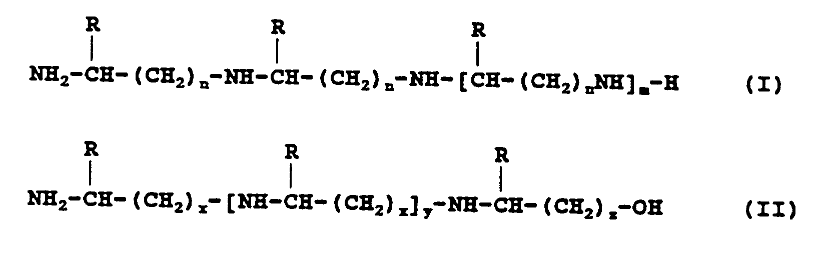 in which R is hydrogen or a methyl group and m, n, x, y and z independently of one another represent 0 or numbers of 1 to 5,
and subsequent neutralization of the reaction products with acids.

2. Salts as claimed in claim 1, characterized in that technical elaidic acid or esters thereof are reacted with amines corresponding to formula **(I)** or **(II)**.

3. Salts as claimed in at least one of claims 1 and 2, characterized in that, in formula **(I)**, R is hydrogen, n is 1 or 2 and m is 0 or 1.

4. Salts as claimed in at least one of claims 1 and 2, characterized in that, in formula **(II)**, R is hydrogen, x and z are 1 and y is 0.

5. A process for the production of salts of fatty acid amides and fatty acid imidazolines, characterized in that unsaturated fatty acids containing 16 to 22 carbon atoms and at least 40 mol-% trans-configured double bonds or esters thereof with glycerol or C₁₋₄ aliphatic alcohols are reacted with amines corresponding to formula **(I)** or **(II)** 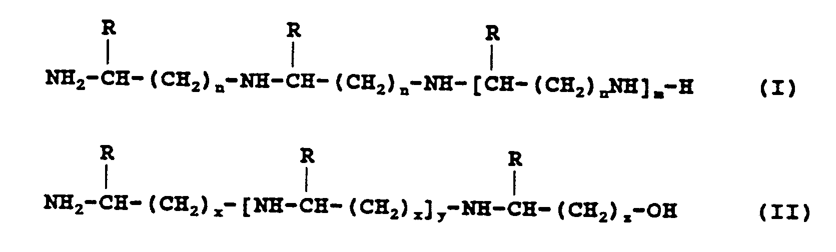 in which R is hydrogen or a methyl group and m, n, x, y and z independently of one another represent 0 or numbers of 1 to 5,
and the reaction products are subsequently neutralized with organic or inorganic acids.

6. A process as claimed in claim 5, characterized in that technical elaidic acid or esters thereof are reacted with amines corresponding to formula **(I)** or **(II)**.

7. A process claimed in at least one of claims 5 and 6, characterized in that amines corresponding to formula **(I)**, in which R is hydrogen, n is 1 or 2 and m is 0 or 1, are used.

8. A process as claimed in at least one of claims 5 and 6, characterized in that amines corresponding to formula **(II)**, in which R is hydrogen, x and z are 1 and y is 0, are used.

9. A process as claimed in at least one of claims 5 to 8, characterized in that the reaction of the unsaturated fatty acids or esters thereof with the amines is carried out in a molar ratio of 1 : 1 to 5 : 1.

10. A process as claimed in at least one of claims 5 to 9, characterized in that the reaction of the unsaturated fatty acids or esters thereof with the amines is carried out at temperatures of 80 to 220°C.

11. A process as claimed in at least one of claims 5 to 10, characterized in that the reaction products are neutralized with acids from the group consisting of phosphoric acid, phosphorous acid, formic acid, acetic acid, glycolic acid and lactic acid.

12. The use of the salts of fatty acid amides and fatty acid imidazolines claimed in any of claims 1 to 4 as fabric softeners.

13. The use of the salts of fatty acid amides and fatty acid imidazolines claimed in at least one of claims 1 to 4 for the production of fabric softeners.

14. The use of the salts of fatty acid amides and fatty acid imidazolines obtainable by the process claimed in at least one of claims 5 to 11 as fabric softeners.

15. The use of salts of fatty acid amides and fatty acid imidazolines obtainable by the process claimed in at least one of claims 5 to 11 for the production of fabric softeners.

## Revendications

1. Sels d'amides d'acides gras et d'imidazolines d'acides gras qu'on obtient par réaction d'acides gras non .saturés ayant de 16 à 22 atomes de carbone et une teneur d'au moins 40 % molaire de doubles liaisons transconfigurées ou de leurs esters avec du glycérol ou des alcools aliphatiques ayant de 1 à 4 atomes de C, avec des amines de formule (I) ou (II). 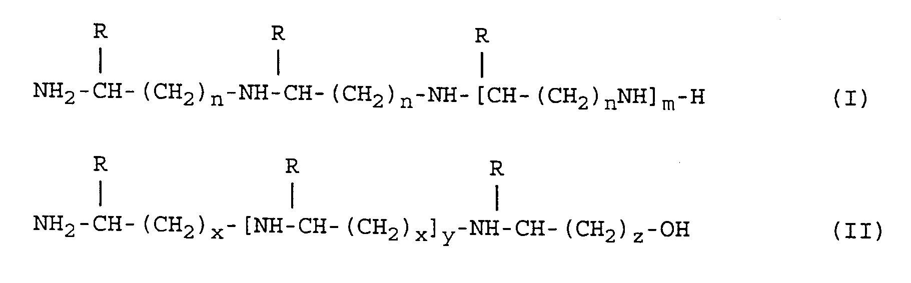 dans lesquelles,
R représente de l'hydrogène ou un radical méthyle ainsi que m, n, x, y et z représentent indépendamment l'un de l'autre O ou des nombres de 1 à 5,
et par neutralisation successive des produits de réaction avec des acides minéraux ou organiques.

2. Sels selon la revendication 1, caractérisés en ce qu'on met en réaction de l'acide élaïdique technique ou ses esters avec des amines des formules (I) ou (II).

3. Sels selon au moins une des revendications 1 et 2, caractérisés en ce que dans la formule (I) R représente de l'hydrogène, n représente 1 ou 2 et m 0 ou 1.

4. Sels selon au moins une des revendications 1 et 2, caractérisés en ce que dans la formule (II) R représente de l'hydrogène, x et z représentent 1 et y 0.

5. Procédé pour la préparation de sels d'amides d'acides gras et d'imidazolines d'acides gras, caractérisé en ce qu'on met en réaction des acides gras ayant de 16 à 22 atomes de carbone et une teneur d'au moins 40 % molaire de liaisons doubles trans-configurées en leurs esters avec du glycérol ou des alcools aliphatiques ayant de 1 à 4 atomes de carbone avec des amines des formules (I) ou (II). 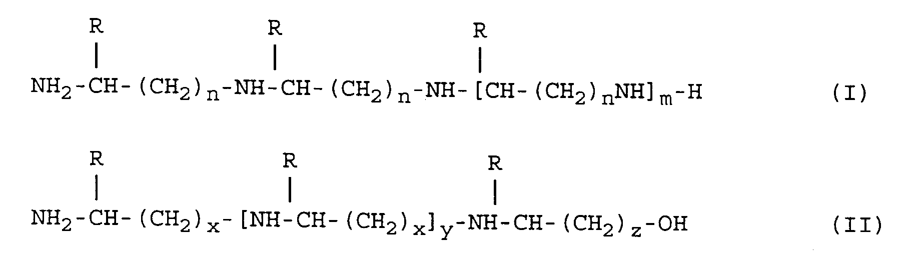 dans lesquelles,
R représente de l'hydrogène ou un radical méthyle ainsi que m, n, x, y et z représentent indépendamment l'un de l'autre O ou des nombres de 1 à 5,
et ensuite on neutralise les produits réactionnels avec des acides minéraux ou organiques.

6. Procédé selon la revendication 5, caractérisé en ce qu'on met à réagir de l'acide élaïdique technique ou ses esters avec des amines des formules (I) ou (II).

7. Procédé selon les revendications 5 et 6, caractérisé en ce qu'on met en oeuvre des amines de formule (I), dans laquelle R représente de l'hydrogène, n représente 1 ou 2 et m 0 ou 1.

8. Procédé selon au moins une des revendications 5 et 6, caractérisé en ce qu'on met en oeuvre des amines de formule (II), dans laquelle R représente de l'hydrogène, x et z représentent 1 et y 0.

9. Procédé selon au moins une des revendications 5 à 8, caractérisé en ce qu'on effectue la réaction des acides gras non saturés ou de leurs esters avec les amines dans un rapport molaire de 1:1 à 5:1.

10. Procédé selon au moins une des revendications 5 à 9, caractérisé en ce qu'on effectue la réaction des acides gras non saturés ou de leurs esters avec des amines à des températures de 80 à 220°C.

11. Procédé selon au moins une des revendications 5 à 10, caractérisé en ce qu'on effectue la neutralisation des produits réactionnels avec des acides du groupe, qui est formé d'acide phosphorique, d'acide phosphoreux, d'acide formique, d'acide acétique, d'acide glycolique et d'acide lactique.

12. Utilisation de sels d'amides d'acides gras et d'imidazolines d'acides gras selon au moins une des revendications 1 à 4 en tant qu'agents d'avivage et agents assouplissants au lavage.

13. Utilisation de sels d'amides d'acides gras et d'imidazolines d'acides gras selon au moins une des revendications 1 à 4 pour préparer des agents d'avivage et des agents assouplissants au lavage.

14. Utilisation de sels d'amides d'acides gras et d'imidazolines d'acides gras, pouvant être obtenus d'après le procédé selon au moins une des revendications 5 à 11 en tant qu'agents assouplissants au lavage.

15. Utilisation de sels d'amides d'acides gras et d'imidazolines d'acides gras, pouvant être obtenus d'après le procédé selon au moins une des revendications 1 à 11 pour préparer des agents d'avivage et des agents assouplissants au lavage.
